# EUROPEAN PATENT SPECIFICATION

(11) **EP 0 639 987 B1**
(45) Date of publication and mention of the grant of the patent: **29.08.2001**
(21) Application number: 94908439.6
(22) Date of filing: 07.03.1994
(51) Int. Cl.: A61L 2/12, A61L 2/24, A61L 2/20

(54) **STERILISING AND DESORBING PROCEDURES AND EQUIPMENT**
VERFAHREN UND VORRICHTUNG ZUR DESORPTION UND STERILISATION
PROCEDURES DE STERILISATION ET DE DESORPTION, ET EQUIPEMENT UTILISE

(30) Priority: 06.03.1993 GB 9304628
(43) Date of publication of application: 01.03.1995
(73) Proprietor: University of Wales College of Medicine, Cardiff CF4 4XN (GB)
(72) Inventor: SAMUEL, Alan Herbert, Whitchurch, Cardiff CF4 7AR (GB); MATTHEWS, Ian Price, Penylan, Cardiff (GB)
(74) Representative: Newell, William Joseph
(86) International application number: GB9400436
(87) International publication number: WO9420150

(56) References cited:
- WO-A-86/02842
- US-A- 3 676 058
- US-A- 3 753 651
- MEDICAL INSTRUMENTATION vol. 22, no. 1 , February 1988 pages 39 - 44 A.H. SAMUEL ET AL. 'MICROWAVE DESORPTION : A COMBINED STERILIZER AERATOR FOR THE ACCELERATED ELIMINATION OF ETHYLENE OXIDE RESIDUES FROM STERILIZED SUPPLIES.'
- THE JOURNAL OF MICROWAVE POWER AND ELECTROMAGNETIC ENERGY. vol. 23, no. 1 , 1988 pages 17 - 28 C. GIBSON ET AL. 'MICROWAVE ENHANCED DIFFUSION IN POLYMERIC MATERIALS.'
- BIOMATERIALS vol. 10 , July 1983 pages 343 - 348 C. GIBSON ET AL. 'COMPUTERIZED MODEL FOR ACCURATE DETERMINATION OF ETHYLENE OXIDE DIFFUSION IN STERILIZED MEDICAL SUPPLIES.'
- AMERICAN JOURNAL OF HOSPITAL PHARMACY vol. 29 , August 1972 pages 661 - 672 R.M.G.BOUCHER 'ADVANCES IN STERILIZATION TECHNIQUES.'
- JOURNAL OF BIOMEDICAL MATERIALS RESEARCH vol. 23, no. 2 , February 1989 pages 143 - 156 I.P. MATTHEWS ET AL. 'ENHANCEMENT OF THE KINETICS OF THE AERATION OF ETHYLENE OXIDE STERILIZED POLYMERS USING MICROWAVE RADIATION.'

## Description

### FIELD OF THE INVENTION

This invention relates to the sterilising of articles to kill or destroy bacteria or other unwanted or toxic bodies. The invention is of particular utility in the sterilising of equipment used for medical or surgical purposes, but may have other applications, for example in industry. This invention also relates to the desorption of gas or vapour from articles or material, and extends in particular to apparatus and methods for measuring the presence or concentration of sterilant, or the gas or vapour to be desorbed in the above sterilising and desorbing techniques. Such apparatus and methods may be used to control sterilising and desorbing apparatus and improve their operation.

### BACKGROUND OF THE INVENTION

It is of course well-known to sterilise metal surgical instruments by steam and it has also been common practice to sterilise various articles in sterilising liquids. High temperature steam sterilising is simple and effective for certain metal objects, but has limitations and disadvantages, for example it is inefficient in sterilising any object within a sealed package, and the high temperatures involved may cause degradation of plastic articles. The use of sterilising liquids also has limitations, for example air bubbles may be trapped within the object thus preventing full effectiveness, and it may be difficult or impossible to remove all trace of the liquid afterwards.

It has also been common practice to sterilise medical or surgical equipment by means of a toxic gas and many existing sterilisers in hospital use, for example, involve contacting the articles with Ethylene Oxide in a sealed sterilising chamber. Ethylene Oxide is an extremely effective bactericide, and is effective not only for metal objects such as surgical implements, but also relatively delicate instruments and equipment, including synthetic plastics. The gas is effective at medium or low temperatures and thus the effects of high temperatures on degrading materials can be avoided. The gas penetrates or permeates plastic wrappings and can thus be used to sterilise prepackaged objects.

Ethylene Oxide is, however, extremely toxic to human beings and after its use for sterilising it is important that steps be taken to remove all traces, as far as possible. To this end it is known to flush out the interior of the sterilising chamber or autoclave with fresh air after the sterilising gas has been extracted. It has also been proposed to "pulse" or vary the pressure of the flushing air during the flushing operation.

One of the problems involved is that the Ethylene Oxide is absorbed into a number of typical plastic materials which may need to be sterilised, and to "desorb" the gas from the plastics appears to be extremely difficult. Even after the sterilising chamber has been flushed the desorption of the gas from the objects may result in contaminating the atmosphere of the laboratory or other workroom to a concentration of, say, 4 ppm or more which is an unacceptable health hazard.

Ethylene Oxide sterilisation is normally validated and monitored by biological measurements. This is achieved by demonstrating the killing of standardised preparations or biological indicators of known resistance to Ethylene Oxide. For routine process monitoring of production cycles after exposure to Ethylene Oxide, the biological indicators are placed in culture media and incubated to determine growth and survival. However, the incubation and inspection of the indicators typically takes several days and so does not allow real-time monitoring of the efficiency of the sterilisation process. Also, whilst indicating whether the sterilising process has been successful it does not indicate whether the concentration of Ethylene Oxide is much higher than necessary (i.e. an "overkill") or only just adequate. Nor can it indicate when it is safe to open the steriliser.

The use of biological monitoring for quality control is less satisfying than parametric monitoring because of the inherent variability of biological monitors and associated recovery restraints.

In Yeung et al (On Line Measurement of the Gaseous Concentration and Relative Humidity in a 100% Ethylene Oxide Sterilisation Cycle J. Parenteral Drug Association, Vol. 33 No.3 pp 117-124) there is proposed a steriliser with a dual column gas chromatograph analyser which samples the atmosphere within the chamber every 90 seconds or so and, using a carrier or transfer gas, conveys the sample through the columns which are usually packed with an inert porous support material whereby the different gaseous components are separated and detected. However, whilst capable of regularly sampling the sterilising chamber atmosphere, a gas chromatograph cannot provide proper real-time measurement of the constituents in the sample because the final measurement is dependent on the slow passage of the sample through the columns.

Also, a characteristic and limiting feature of a gas chromatography analyser is that the columns cannot be sterilised, or more correctly, if the columns were sterilised it would render them useless for the purpose of measurement.

Furthermore, the act of sampling breaches the all-important sterility or barrier boundary after sterile conditions have been achieved, thus compromising the sterilisation procedure.

Yeung et al suggest that, to prevent the carrier gas from contaminating (either chemically or biologically) the sterilising chamber, the samples are vented to waste rather than returned to the chamber. This inevitably creates a disposal problem and necessarily alters the in-chamber conditions being measured. Furthermore since there is no constant flow path from the steriliser to the analyser and back to the steriliser, this further frustrates any attempt to obtain measurements in real-time.

WO-A-86/02842 discloses a microwave steriliser which includes a microwave spectrometer for detecting or measuring ethylene oxide.

It is an object of this invention to provide an improved steriliser or desorber in which the presence or concentration of a gas or vapour can be detected substantially in real-time, so that the sterilisation or desorption process can be automatically controlled on the basis of these measurements.

It is a further object of this invention to provide a steriliser or desorber having a sampling means for determining the presence and/or concentration of a gas or vapour, which is capable of sterilisation.

Accordingly, in one aspect this invention provides a sterilising and/or desorbing apparatus including:-
a treatment chamber;
means for releasing a sterilising gas or vapour into said treatment chamber;
means for removing said sterilising gas or vapour from said treatment chamber;
sampling means for sampling the atmosphere within said treatment chamber, and including:-
   means for exposing said atmosphere sample to microwave radiation, and
   means responsive to the absorption of microwave radiation by said sample to determine the presence of and/or concentration of gas or vapour in said sample;
   wherein said apparatus further includes control means responsive to said determined presence and/or concentration of gas or vapour to control at least one of said releasing means and said removing means.

Preferably, said sterilising and/or desorbing apparatus further includes passage means connecting said treatment chamber and said sampling means, and through which atmosphere may pass from the treatment chamber to said sampling means.

Preferably said passage means includes means for causing atmosphere from said treatment chamber to circulate through said sampling means.

In one embodiment, said sterilising apparatus includes lock means and said control means is operable to allow said treatment chamber to be opened only when the concentration of said gas or vapour is below a given level, as determined by said sampling means.

Said control means may additionally be operable to control said release means in response to said sampling means to control the amount of gas or vapour released into said treatment chamber, thereby to limit the concentration of said gas or vapour in said chamber.

Said control means may furthermore be operable to monitor the concentration of said gas or vapour in said treatment chamber during said desorption step and control said propagating means to control the period of irradiation in accordance with the sensed concentration of said gas or vapour.

Said sampling means may include a sensing chamber into which a sample of said chamber atmosphere is introduced, means for propagating microwave radiation in the sensing chamber and means for varying the quantum mechanical molecular rotational frequencies of the analyte, the frequency of the microwave source and the resonant frequency of the sensing chamber such that they are in sympathy and thereby determining the presence and concentration of said gas or vapour in said chamber.

In another aspect, this invention provides a method of sterilising and/or desorbing articles, said method including:-
placing said articles in a treatment chamber;
releasing a sterilising gas or vapour into said treatment chamber;
thereafter removing at least some of said sterilising gas or vapour from said treatment chamber sampling the atmosphere within said treatment chamber by exposing an atmosphere sample to microwave radiation and determining the pressure and/or concentration of gas and vapour in said sample based upon absorption of microwave radiation by said sample,
wherein said method further includes controlling at least one of said releasing and said removing in accordance with the determined presence and/or concentration of said gas or vapour.

By this arrangement, the presence and concentration of Ethylene Oxide may be measured in real-time, in a mixture which might contain several constituents, e.g. water vapour, vapours given off by the articles being sterilised, and so on. This means that, in contrast to earlier arrangements, the ability of the sampling means to identify and accurately quantify a particular gas or vapour allows the sampling means to form part of an active control loop. This means that it may no longer be necessary to supply an excessive concentration of sterilant to the steriliser to provide a generous margin of error. Thus the concentrations of steriliser used may be considerably reduced, with a corresponding reduction of the amount of time necessary to desorb harmful sterilants and a removal or reduction of the disposal problem. It also allows the sterilant to be recycled because an accurate measure of its concentration can be taken.

Furthermore, the sampling means provides a sensing chamber within which the sample is exposed to radiation and this may easily be sterilised as compared to the non-sterilisable column(s) of the gas chromatograph.

The steriliser described herein makes use of our said sample.

In a further aspect, the invention provides a desorbing apparatus, including a desorbing chamber, means for sampling the atmosphere within said desorbing chamber, said sampling means including means for exposing said atmosphere sample to microwave radiation and means responsive to the absorption of microwave radiation by said sample to determine the presence and concentration of gas or vapour in said sample.

In a further aspect, the invention provides a method of sterilising an article or material which comprises monitoring the presence or concentration of a gas or vapour in the sterilising chamber by exposing a sample of said chamber atmosphere to microwave radiation and determining the presence or concentration of a selected gas or vapour by observing the absorption of said radiation at a frequency or frequencies corresponding to said selected gas or vapour.

In yet another aspect, the invention provides a method of desorbing gas or vapour from an article or material which comprises exposing said article or material to high frequency electromagnetic radiation in a desorbing chamber and monitoring the presence or concentration of a gas or vapour in the desorbing chamber by exposing a sample of said chamber atmosphere to microwave radiation and determining the presence or concentration of a selected gas or vapour by observing the absorption of said radiation at a frequency or frequencies corresponding to said selected gas or vapour.

The steriliser described herein makes use of our discovery that microwave energy has important and surprising advantages in the sterilising field. As will be explained more fully it has a valuable effect in aiding the desorption of a gas such as Ethylene Oxide, and it also has a valuable synergistic effect in the actual sterilising process when used in conjunction with Ethylene Oxide. More particularly the invention is based in part on the discovery that microwave energy exerts a non-thermal as well as a thermal effect upon materials, and the synergistic use of microwave radiation in conjunction with Ethylene Oxide gas for sterilization is far more effective than use of either agent alone. This is believed to follow from two allied but different reasons.

Non-thermal effects result directly from the interaction of the electromagnetic field with molecules. When polar molecules are subjected to a strong alternating electromagnetic field their tendency to orientate with the applied field causes a degree of molecular disturbance. Thermal effects, on the other hand, are a consequence of "dielectric loss" in which part of the applied field voltage is resolved as Joule heating due to the inability of the molecular dipole to align with the field with absolute fidelity, i.e. the molecular orientations are slightly out of phase with the applied field due to hindered movement. It has been found that microwaves exert a biocidal effect distinct from that of thermal energy, and it is believed that this may result from their effect on metabolic chemistry, possibly by causing alternations in cell membrane permeability, or by altering the pH (acidity) of the cellular environment, or by modifying weak chemical bonds between cellular macro molecules. Importantly also it is believed that a very advantageous non-thermal biocidal effect of microwaves lies in the degree of absorption of microwave energy by key molecules such as deoxyribonucleic acid (DNA). For these reasons it is believed that microwave irradiated micro-organisms are less viable and more susceptible to destruction by ethylene oxide gas. A mode of action involving direct effect upon cellular DNA particularly favours the synergistic use with ethylene oxide gas, which also exerts a direct effect upon DNA.

The biocidal efficacy of ethylene oxide is a consequence of its electrophilic and lipophilic nature and the reactivity of the epoxide ring. It combines very readily with nucleophilic centres on nucleic acids and proteins. The mechanism of DNA alkylation by ethylene oxide is principally at the site of the N₇ - atom of the base guanine leading to N₇ - (2-hydroxyethyl) guanine. It is believed that the interaction of the applied microwave electric field with the bonding electrons in molecules of ethylene oxide may induce further significant polarization of bonds, thus enhancing the electrophilic reactivity of the molecule.

For sterilization purposes ethylene oxide gas has sometimes been mixed with freon or carbon dioxide in the proportion of for example 12% E.O. and 88% freon or CO₂. In performing the present invention it is possible to use the gas to transfer heat from the material to be sterilized by recycling and compressing the gas (as in a conventional refrigeration process). This may permit a much greater input of microwave energy into the materials thereby enhancing the non-thermal effects whilst maintaining materials at an acceptable temperature.

A preferred embodiment provides sterilizing apparatus comprising a chamber having an access opening an a closure therefore, fluid-tight sealing means for the closure, means for introducing a controlled gas into the chamber and for extracting the gas when required, means for generating or radiating very high frequency electro-magnetic radiation in or into the chamber, and means to limit or prevent unwanted escape or discharge of the radiated energy.

Preferably the closure seal is also provided with means to restrict or prevent escape of electro-magnetic radiation and the apparatus also preferably includes a movable support for objects to be sterilised within the chamber, and drive means operable from outside the chamber for actuating the support. The drive means may include a drive coupling between the interior and exterior of the chamber, the coupling being arranged to maintain the positive fluid and radiation seals.

According to another preferred feature of the invention the apparatus includes means to locate a sealed container for the gas or liquid within the chamber and means to discharge the fluid from the container within the chamber, the actuating means being operable from outside the chamber without disturbing the gas or radiation seals.

The radiation generated may be a microwave heating system arranged to create or introduce microwave radiation into the chamber through a sealed wall, "transparent" to the microwave radiation,and the generator may also include means for varying the level of radiation.

Preferably the apparatus includes means for varying or controlling the pressure within the chamber, e.g. above or below atmospheric.

From another aspect the invention consists in a method of sterilising an article in which the article is placed in a sealed chamber, sterilising gas is introduced into the chamber and later withdrawn, high frequency electro-magnetic radiation is created or introduced into the chamber to cause internal heating of the article and/or any adsorbed gas, and hence desorption of the gas, and the article is then withdrawn from the chamber.

The article is conveniently introduced into and withdrawn from the chamber through a closure which includes a fluid-tight seal and a radiation seal.

The gas is conveniently Ethylene Oxide, which is extremely toxic to human beings and preferably the sterilising gas, or liquid, is initially contained in a sealed capsule or container placed within the chamber, and the gas is released from the container by an actuator operated externally of the chamber.

Conventional thermometers are not effective in a microwave field and according to another preferred feature of the invention the temperature within the chamber, or of the article, is sensed from outside the chamber by an infra-red pyrometer or other remote thermal sensor unaffected by the radiation.

The microwave radiation will normally be created in the chamber after the sterilising gas has been withdrawn so as to encourage desorption of the gas into the surrounding air or other atmosphere. It may, however, in some cases be of advantage to introduce or create the radiation in the chamber while the sterilising gas is within the chamber and this may assist the sterilising process.

In any case the article to be sterilised is preferably moved or rotated within the chamber during exposure to the gas and/or the radiation by means of a motivating system actuated externally.

The invention may also be applied as an additional feature to existing sterilising equipment and procedures. Thus from another aspect the invention also counts in gas desorbing apparatus for use in conjunction with gas sterilising apparatus, comprising a fluid tight chamber having, an access opening and a closure and fluid tight sealing means for the closure, means for introducing a cleansing or flushing gas such as air into the chamber and for extracting the said gas, means for generating or radiating high-frequency electro-magnetic radiation in or into the chamber, and means to limit or prevent unwanted escape or discharge of the radiated energy.

Likewise the invention also consists in a method of desorbing gas absorbed into an article for sterilisation, in which the article is placed in a sealed desorbing chamber, high-frequency electromagnetic radiation is created in or introduced into the chamber to cause internal heating of the article and/or any absorbed gas, and hence desorption of the gas, and the article is then removed from the chamber.

According to a preferred feature of the invention the apparatus may include automatic or semi-automatic systems for controlling the cycle, comprising one or more sensers for detecting selected parameters of the operating conditions such as chamber pressure, or temperature, radiation level, concentration of gas, and physical state of actuating elements, together with a programmed controller, and automatic control function elements actuated by the controller for performing selected steps in the cycle.

The invention may be performed in various ways and one specific embodiment will now be described by way of example with reference to the accompanying drawings, in which:-
Figure 1 is a diagrammatic side elevation of a gas sterilising apparatus according to the invention,
Figure 2 is a diagrammatic plan view of the apparatus,
Figure 3 is a diagrammatic side elevation on an enlarged scale illustrating the drive mechanism and coupling for rotating the internal support basket,
Figure 4 is a diagrammatic view illustrating two components of the drive coupling,
Figure 5 is a diagrammatic side elevation on an enlarged scale illustrating the sealed internal gas release device,
Figure 6 is a diagrammatic end view illustrating the housing for the capsule puncturing needle,
Figure 7 is a diagram illustrating a microwave spectrometer for detecting Ethylene Oxide in the equipment,
Figure 8 is a diagram illustrating components of a microwave spectrometer system for use in performing the invention, and
Figure 9 is a diagram illustrating a general automatic control system for a sterilising/ desorbing cycle.

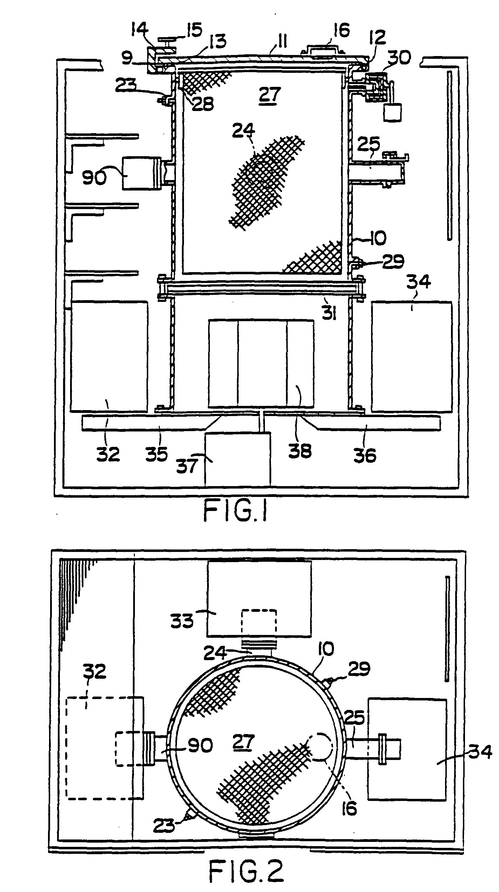

Referring first to the essential elements of the apparatus illustrated in Figures 1 and 2, the equipment comprises a sterilising chamber 10 having a stainless steel cylindrical side wall designed to withstand internal pressures, for example of up to five atmospheres, or a partial vacuum. The top of the chamber is closed by a circular lid or cap 11, which is hinged to the side wall at 12 and provided with a circumferential O-ring or other fluid seal 13, and a metallic knit-mesh microwave seal 9. The lid can be held closed by a hinged clamp 14 provided with a tightening screw 15. The lid may also have a rupturable pressure relief disc valve 16, to prevent build up of excessive internal pressures.

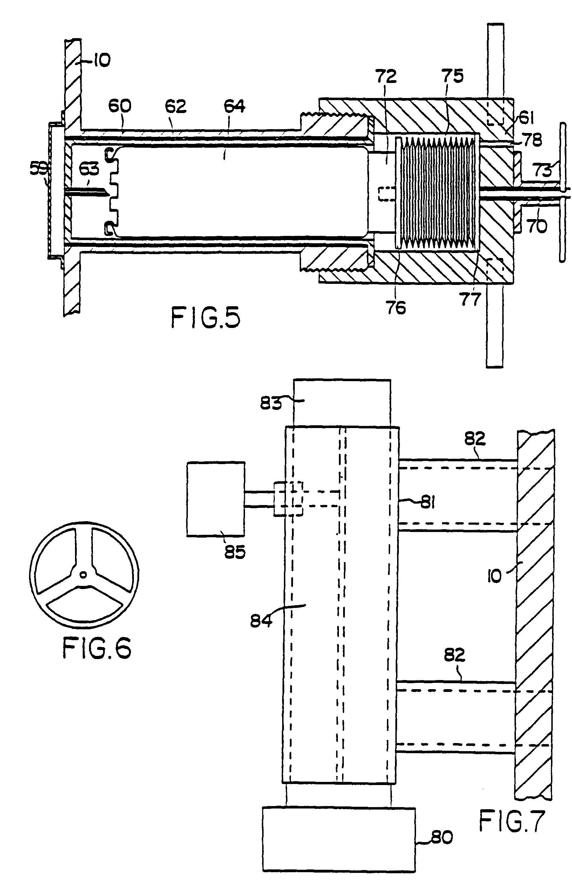

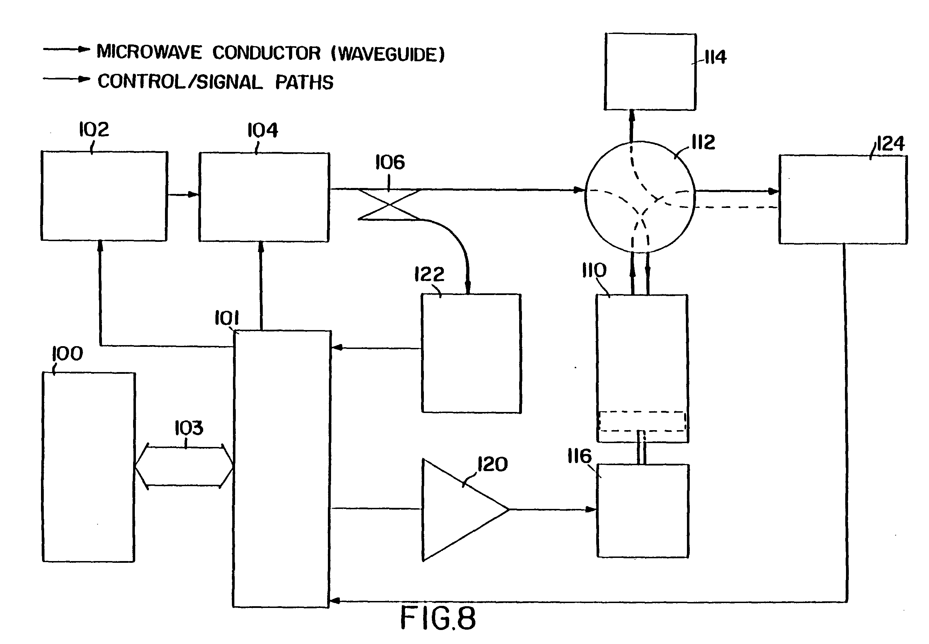

On the side of the chamber 10 is an air entry 23 which may be connected to a source of supply for a controlled selected gas, or atmosphere, as required. The side wall of the chamber is also provided with another port 29 intended to act as an air outlet, and this may be connected to a special vent duct with a filter, or to atmosphere, as required. The filter conveniently includes a mass of high molecular weight polymeric material designed to adsorb Ethylene Oxide, from which the gas can later be reclaimed for disposal or recycling. The wall of the chamber is also provided with a microwave spectrometer 24, as illustrated in Figure 7, and at another part of the side wall is a special sealed injector unit 25 for admitting controlled quantities of Ethylene Oxide when required. This is illustrated in greater detail in Figures 5 and 6.

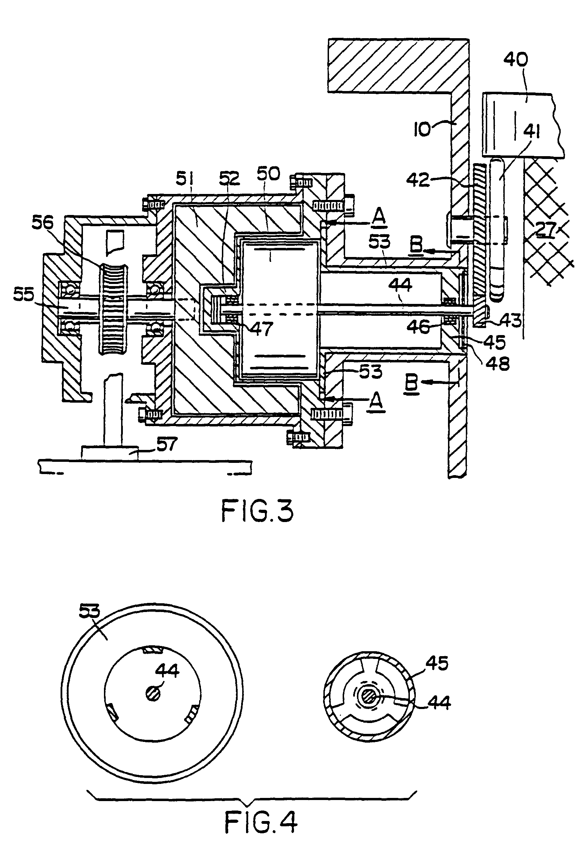

Within the chamber is a rotary basket 27 for equipment to be sterilised, the basket being supported by bearings 28 mounted on the side wall of a chamber and a special drive system 30 for causing rotation of the basket is illustrated in detail in Figures 3 and 4. The bottom wall 31 of the chamber is formed of a material such as Polypropylene - which is capable of transmitting electromagnetic radiation and acts as a microwave window, and below the chamber are located three microwave generators 32,33,34 associated with launching "horns" 35,36 and a "mode stirrer" 38 driven by a motor 37. Means are provided to vary the output of the generator 33 and to augment this as required by one or both of the generators 32,34 so as to provide for variation over all three ranges.

The drive mechanism for the basket 27, as illustrated in Figures 3 and 4, is arranged to provide for effective drive coupling without disturbing the microwave and fluid seals. As illustrated, the basket 27 has an upper rim 40 which is supported on the free bearing rollers 28 and at one point on a driven roller 41 within the chamber wall 10. This roller 41 is connected to a pinion 42 coupled to a smaller pinion 43 on a shaft 44 which passes through an opening in a metallic mesh microwave screen 48, and through an aperture in the wall 45, and is supported by bearings 46,47. Also mounted on the shaft 44 is a magnetic driven rotor 50 which is closely associated with a driving magnet 51, but sealed therefrom totally by an intervening wall 52 which acts as a positive fluid seal or enclosure and is in permanent static contact with a cylindrical extension 53 of the chamber wall 10. The magnet 51 is mounted on a drive shaft 55 supported in bearings and secured to a gear 56 forming part of a worm drive coupled to a driving motor 57. Figure 4 illustrates cross-sections through the coupling unit on the lines A-A and B-B in Figure 3.

Thus it will be seen that control movements imparted by the motor 57 cause rotation of the magnet 51 and hence of the driven rotor 50 and the basket drive roller 41. In this way the basket with its contents can be moved continuously or in intervals as required for optimum sterilisation.

The Ethylene Oxide injector illustrated in Figures 5 and 6 is arranged to be totally sealed within the chamber 10 but can be actuated externally. The wall 10 of the chamber has an extension 60 to which is positively attached in a fluid tight manner a cylindrical housing 61 and between the two is located a sleeve 62 having a sharp pointed hollow needle 63 at its inner end arranged when required to perforate a sealed capsule 64 containing liquid Ethylene Oxide. When punctured the liquid contents pass through the needle, impinge upon a baffle plate 59,and are rapidly vapourised into the interior of the chamber. The capsule is forced against the needle by means of an abutment plate 72 which is urged towards the outer end of the capsule by a metal bellows 75 expanded by the pressure of atmospheric air entering the internal void of the bellows via a port 78. The injector unit is permanently sealed off from the atmosphere by the bellows which is closed at its inner end and positively sealed at its outer end to an annulus 77 secured within the cylindrical housing 61. The abutment 72 is provided with a shaft 70 which passes through the void within the bellows and thence through the annulus and the end of the housing 61. The shaft is provided with a manual locking lever which can be pulled outwards and rotated to lock the shaft thus holding the bellows in the contracted position. Evacuation of the main chamber causes the bellows to experience a pressure equal to the difference between atmospheric pressure and chamber pressure. When the shaft is released the pressure differential causes air to enter the internal void of the bellows via the port 78 extending the bellows and driving the abutment plate inwards. For safety reasons this arrangement provides a means of puncturing the Ethylene Oxide canisters which will only operate when the chamber pressure is reduced below atmospheric pressure.

Figure 7 illustrates a microwave molecular rotational spectrometer attached to the chamber 10 for detecting the presence and concentration level of Ethylene Oxide and other gases. Molecular rotational microwave spectrometry is a fundamental analytical technique. Operating on the principle of quantised absorption of electromagnetic radiation, a molecular dipole in the gas phase will exhibit promotion of its rotational state to one of many available energy levels resulting in a highly characteristic narrow bandwith (absorption) spectrum unique to that molecular species; reflecting as it does properties of the entire molecule rather than those of just the nucleus or constituent atoms or groups. Furthermore the magnitude of absorption maxima at resonant frequencies is related to the molecular concentration of the analyte and at low pressures when molecular interactions are at a minimum a linear concentration relationship is established. Absorption "peaks" for many compounds may be detected at frequencies between 10 Gigahertz and 40 Gigahertz. The spectrometer illustrated in Figure 7 is comprised of a rectangular wave guide tube 81 having a Klystron, Gunn diode or other suitable low power monochromatic microwave generator 80 at the lower end and a microwave detector 83 at the upper end. The interior of the tube is connected to and communicates with the interior of the main chamber 10 via two rigid welded branch tubes 82 each equipped with a microwave choke to prevent the escape of microwave energy while permitting the free diffusion of gases and vapours via suitable permeable septa. Within the tube 81 is a thin insulated metal strip electrode positioned centrally along the major axis of the tube. This electrode is connected to an external alternating square wave voltage source such that a potential difference arises between the electrode and the tube during every half of the alternating voltage cycle giving rise to "Stark" splitting of the angular momentum of the molecular dipoles and a corresponding shift in the frequency of the microwave absorption for the particular molecular rotational transition under observation. The microwave detector passes signals to a detector amplifier which incorporates a phase sensitive discriminator operating synchronously with the "Stark" modulated voltage. The detector amplifier output thus reflects microwave energy absorption occurring each half cycle during zero electrode potential. This arrangement improves sensitivity by improving the signal to noise ratio. According to another preferred feature where greater spectrometer sensitivity is required the waveguide tube 81 may be replaced by a microwave resonant cavity of high quality (Q) factor connected to and communicating with the interior of the main chamber as previously described and having a monochromatic microwave generator and a microwave detector at appropriate positions and a central electrode with which to employ the "Stark" effect.

The pressure of the gas sample in the spectrometer is approximately 20 millitorr (2.7 Pa) maintained by a high vacuum pump. Gas molecules diffuse into the spectrometer through permeable septa.

According to yet another preferred feature a microwave rotational spectrometer utilising multiple sequential scanning as a means of signal enhancement which obviates the need for Stark modulation voltages may be used. Figure 8 is a schematic diagram of such a spectrometer control system for use in controlling the process of ethylene oxide sterilisation. In this system a microprocessor unit 100 is arranged to control automatically the operation of the instrument and analyse the data received from the power measuring devices. An interfacing unit 101 includes electronic circuitry to link the microprocessor data bus 103 to the remainder of the spectrometer components. A Gunn Diode oscillator 102, under the control of the microprocessor is arranged to "search" for a specific absorption peak by sweeping a preselected frequency band. The power output from the oscillator is limited by means of a precision attenuator 104. A directive coupling 106 splits the microwave beam from the Gunn Oscillator into two halves so that the power incident upon the gas filled cavity 110 may be continuously monitored by sensing one half of the beam. A waveguide circulator 112 enables the other half of the microwave beam to be fed into/out of the same cavity orifice. This avoids the possibility of interference between the incident and transmitted beams which would adversely affect the accuracy of measurement. A power absorbing load 114 soaks up any microwave power reflected from the measuring devices.

The tunable resonant cavity 110 has its resonant frequency varied electromechanically in order to match it to the frequency of the input radiation. As a result, at resonance the microwave beam will pass through the comparatively small volume of gas very many times, thus greatly increasing the efficiency of the absorption.

The necessary minute changes in cavity geometry are achieved with the desired degree of precision by means of a piezoelectric positioning device 116. This converts changes in an applied voltage into changes in mechanical displacement (up to 100 microns). The device 116 is powered by an amplifier 120 controlled from the microprocessor interfacing unit 101, and there are two power measuring devices 122,124 also associated with the interfacing.

The equipment also includes an infra-red remote pyrometer, illustrated diagrammatically at 90, on the side wall of the chamber 10, which enables temperature readings to be obtained within the chamber without affecting or being affected by the microwave field.

The equipment may also include an internal pressure sensor designed to avoid interference with the microwave field and arranged not to affect the positive sealing of the chamber.

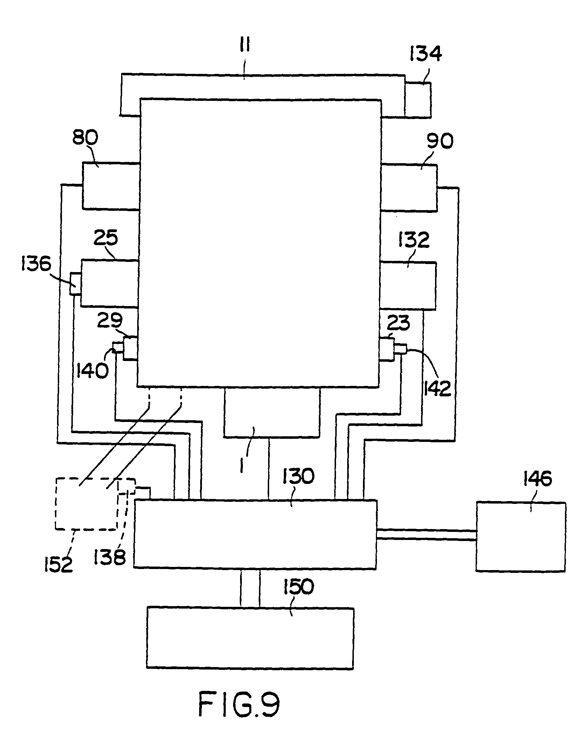

The whole equipment is conveniently controlled by an automatic or semi-automatic control system, as illustrated diagrammatically in Figure 9. One possible layout includes a central microprocessor unit 130 having a number of inputs from sensors associated with the equipment, and output functions which may be fully automatic or may be in the form of indicators for use by an operator. Amongst other possible sensors forming part of the control is the spectrometer 80-85, as illustrated in Figure 7, and in addition the infra-red pyrometer 90, or other temperature sensor, a vacuum gauge or pressure sensor 132 for the interior of the chamber, and a number of safety micro switches, for example switch 134, to indicate that the cover 11 is safely closed and fluid-tight, switch 136 to show that the ethylene oxide injector 25 is also closed, switch 138 to show that the vacuum pump is or is not operating, and switches 140,142 to show that the air vent 29 or the air inlet 23 is open or closed. In addition, there is a multiple function timer 146 providing both a fractional time count for individual steps in the process and an overall time measurement. The microprocessor outputs provide control of solenoid operated valves and other control functions via an interface 150. These automatically controlled function elements include valves or actuators associated with the ethylene oxide injector 25, the air inlet and vent 23,29, the vacuum pump 152, and the microwave generators 32,33,34.

The preferred "duty cycle" for the equipment is as follows:-
(a) Pre-treatment of the objects to be sterilised including partial evacuation of air from the chamber combined with a humidifying step by addition of water vapour to ensure that bacteria spores and vegetative forms are more susceptible to Ethylene Oxide. This may be followed by a flushing out of the chamber with other gases, for example CO₂.
(b) The Sterilising Staae.
   Ethylene Oxide is admitted under subatmospheric partial vacuum conditions and sterilisation continues either for a fixed time or until predetermined parameters are reached as determined by the sensors on the equipment. During this sterilisation the internal pressure in the chamber returns nearly to atmospheric as a result of the injection of Ethylene Oxide.
(c) Flushing.
   The Ethylene Oxide is pumped out of the chamber and the pressure reduced to a substantial vacuum. Flushing air is then admitted and the chamber again evacuated and pumped down to a lower vacuum. This pulsing may continue through several cycles.
(d) With the chamber pressure at some pre-determined pressure below atmospheric the microwave generator is actuated and allowed to run for a period of time, which may be set by an automatic timer or determined by readings obtained from the sensors particularly the microwave spectrometer which gives an indication of the Ethylene Oxide remnant in the chamber. At the end of the microwave stage the chamber is again pumped out and there may be a further flushing step with clean air. Provided that the parameters are then within the specified ranges, particularly the reading of the spectrometer, the cycle terminates and the cover is automatically unlocked.

The arrangement described above provides a steriliser or a desorber with a sensor which detects the presence and/or concentration of a gas or vapour and which can itself be sterilised. Furthermore, the sensor can be operated with its measuring cell as an integral part of the steriliser chamber without any non-sterilisable interface intervening, thus assuring preservation of sterile conditions during the sterilisation process.

Also, microwave rotational spectrometry as used here provides a primary measurement (i.e. one dependent on a fundamental constant of the analyte specimen) and does not need to be related to a secondary standard, as for example is required in gas chromatography.

This technique of quantitative measurement of gases by microwave rotational spectrometry relies on the fact that the molecular system will absorb energy at some very sharply defined frequencies across the microwave spectrum. Thus this technique relies solely upon the intervention of microwave radiation with the molecular species of interest and consequently yields a primary measurement of the number of molecules of the species of interest. The technique allows the unequivocal measurement of a particular gas or vapour in a complex mixture of unknown compounds and also the ability to identify and quantify the appearance of unexpected spectral features relation possibly to "alien" analyte species.

The specificity of the technique is very high and the probability of overlap between lines of different species even in gas mixtures of very many component gases is very low. The quantitative analysis by microwave spectrometry does not require the use of standards and furthermore allows measurements to be taken over a wide dynamic range (1 to 10⁶ ppm).

## Claims

1. A sterilising and/or desorbing apparatus including:-
a treatment chamber (10);
means for releasing a sterilising gas or vapour into said treatment chamber (10);
means (29) for removing said sterilising gas or vapour from said treatment chamber (10);
sampling means (24/81) for sampling the atmosphere within said treatment chamber (10), and including:-
means (80,81) for exposing said atmosphere sample to microwave radiation, and
means (83) responsive to the absorption of microwave radiation by said sample to determine the presence of and/or concentration of gas or vapour in said sample;
**characterised in that** said apparatus further includes control means (130) responsive to said determined presence and/or concentration of gas or vapour to control at least one of said releasing means and said removing means.

2. A sterilising and/or desorbing apparatus according to Claim 1, further including passage means (82) connecting said treatment chamber (10) and said sampling means (24) and through which atmosphere may pass from the treatment chamber (10) to said sampling means (24).

3. A sterilising and/or desorbing apparatus according to Claim 2, wherein said passage means (82) includes means for causing atmosphere from said treatment chamber (10) to circulate through said sampling means (24).

4. A sterilising and/or desorbing apparatus according to any of the preceding claims, wherein said sterilising apparatus includes lock means and said control means (130) is operable to allow said treatment chamber to be opened only when the concentration of said gas or vapour is below a given level, as determined by said sampling means.

5. A sterilising and/or desorbing apparatus according to any of the preceding claims, wherein said control means is operable to control said release means in response to said sampling means to control the amount of gas or vapour released into said treatment chamber, thereby to limit the concentration of said gas or vapour in said treatment chamber.

6. A sterilising and/or desorbing apparatus according to any of the preceding claims wherein said control means is operable to monitor the concentration of said gas or vapour in said treatment chamber during said desorption step, and to control said propagating means to control the period of irradiation in accordance with the sensed concentration of said gas or vapour.

7. A sterilising and/or desorbing apparatus according to any of the preceding claims, wherein said sampling means includes a sensing chamber into which a sample of said chamber atmosphere is introduced, means for propagating microwave radiation in the sensing chamber and means for varying the quantum mechanical molecular rotational frequencies of the analyte, the frequency of the microwave source and the resonant frequency of the sensing chamber such that they are in sympathy and thereby determining the presence and/or concentration of said gas or vapour in said treatment chamber.

8. A method of sterilising and/or desorbing articles said method including:-
placing said articles in a treatment chamber (10);
releasing a sterilising gas or vapour into said treatment chamber (10);
thereafter removing at least some of said sterilising gas or vapour from said treatment chamber (10), and
sampling the atmosphere within said treatment chamber (10) by exposing an atmosphere sample to microwave radiation and determining the presence and/or concentration of gas and vapour in said sample based upon absorption of microwave radiation by said sample,
**characterised in that** said method further includes controlling at least one of said releasing and said removing in accordance with the determined presence and/or concentration of said gas or vapour.

9. A method according to claim 8, which further includes causing atmosphere from said treatment chamber (10) to circulate through said sampling means (24).

10. A method according to Claim 8 or 9, which further includes preventing access to said treatment chamber (10) until the concentration of said gas or vapour is below a preset level.

## Patentansprüche

1. Vorrichtung zum Sterilisieren und/oder Desorbieren, folgendes umfassend:
eine Prozeßkammer (10);
Mittel zum Freigeben eines Sterilisierungsgases oder -dampfes in die Prozeßkammer (10);
Mittel (29) zum Entfernen des Sterilisierungsgases oder -dampfes aus der Prozeßkammer (10);
einen Probenehmer (24/81) zum Entnehmen einer Probe der Atmosphäre innerhalb der Prozeßkammer (10), folgendes umfassend:
Mittel (80,81) zum Aussetzen der Probe der Atmosphäre einer Mikrowellenstrahlung;
Mittel (83), welche auf die Absorption von Mikrowellenstrahlung durch die Probe ansprechen, um das Vorhandensein und/oder die Konzentration von Gas oder Dampf in der Probe festzustellen;
**dadurch gekennzeichnet,**
daß die Vorrichtung ferner Steuermittel (130) umfaßt, welche auf das festgestellte Vorhandensein und/oder die festgestellte Konzentration von Gas oder Dampf ansprechen um wenigstens eines der Mittel zum Freigeben und der Mittels zum Entfernen anzusteuern.

2. Vorrichtung zum Sterilisieren und/oder Desorbieren nach Anspruch 1, **dadurch gekennzeichnet,** daß zum Verbinden der Prozeßkammer (10) mit dem Probenehmer (24) Leitungsmittel (82) vorgesehen sind, welche zum Durchleiten von Atmosphäre aus der Prozeßkammer (10) zum Probenehmer (24) ausgebildet und angeordnet sind.

3. Vorrichtung zum Sterilisieren und/oder Desorbieren nach Anspruch 2, **dadurch gekennzeichnet,** daß die Leitungsmittel (82) Mittel zum Herbeiführen einer Zirkulation der Atmosphäre aus der Prozeßkammer (10) durch den Probenehmer (24) umfassen.

4. Vorrichtung zum Sterilisieren und/oder Desorbieren nach wenigstens einem der vorhergehenden Ansprüche, **dadurch gekennzeichnet,** daß die Vorrichtung zum Sterilisieren Verriegelungsmittel umfaßt, wobei die Steuermittel (130) derart arbeiten, daß ein Öffnen der Prozeßkammer (10) nur möglich ist, wenn die Konzentration des Gases oder Dampfes unterhalb eines von dem Probenehmer festgestellten, vorbestimmten Wertes liegt.

5. Vorrichtung zum Sterilisieren und/oder Desorbieren nach wenigstens einem der vorhergehenden Ansprüche, **dadurch gekennzeichnet,** daß die Steuermittel derart arbeiten, daß sie die Mittel zum Freigeben in Abhängigkeit von dem Probenehmer steuern, um die Menge des in die Prozeßkammer freigegebenen Gases oder Dampfes zu steuern und dadurch die Konzentration des Gases oder Dampfes innerhalb der Prozeßkammer zu begrenzen.

6. Vorrichtung zum Sterilisieren und/oder Desorbieren nach wenigstens einem der vorhergehenden Ansprüche, **dadurch gekennzeichnet**, daß die Steuermittel derart arbeiten, daß sie die Konzentration des Gases oder Dampfes in der Prozeßkammer während des Desoptionsschrittes überwachen und das Ausbreitungsmittel gemäß der gemessenen Konzentration des Gases oder Dampfes steuern.

7. Vorrichtung zum Sterilisieren und/oder Desorbieren nach wenigstens einem der vorhergehenden Ansprüche, **dadurch gekennzeichnet**, daß der Probenehmer folgendes umfaßt, eine Meßkammer, in welcher eine Probe der Kammeratmosphäre eingeführt wird, Mittel zum Abstrahlen von Mikrowellenstrahlung in die Meßkammer und Mittel zum Verändern der quantenmechanischen Molekülrotationsfrequenz der Analysensubstanz, der Frequenz der Mikrowellenstrahlungsquelle und der Resonanzfrequenz der Meßkammer derart, daß dieses in Resonanz sind, zum Bestimmten des Vorhandenseins und/oder der Konzentration des Gases oder Dampfes in der Prozeßkammer.

8. Verfahren zum Sterilisieren und/oder Desorbieren von Gegenständen mit folgenden Schritten:
Plazieren der Gegenstände in einer Prozeßkammer (10);
Freigeben eines Sterilisationsgases oder -dampfes in die Prozeßkammer (10);
anschließendes Entfernen wenigstens eines Teils des Sterilisationsgases oder -dampfes aus der Prozeßkammer (10) und
Abtasten der Atmosphäre innerhalb der Prozeßkammer (10) durch Bestrahlen einer Probe der Atmosphäre mit Mikrowellen und Bestimmen des Vorhandenseins und/oder der Konzentration von Gas und Dampf in der Probe auf der Basis von Absorption von Mikrowellenstrahlung durch die Probe,
**dadurch gekennzeichnet,**
daß zusätzlich das Freigeben und/oder Entfernen gemäß des gemessenen Vorhandenseins und/oder gemäß der gemessenen Konzentration von Gas oder Dampf gesteuert wird.

9. Verfahren nach Anspruch 8, **dadurch gekennzeichnet,** daß die Atmosphäre innerhalb der Prozeßkammer (10) durch den Probenehmer (24) zirkuliert wird.

10. Verfahren nach Anspruch 8 oder 9, **dadurch gekennzeichnet,** daß ein Zugang zur Prozeßkammer (10) unterbunden wird, bis die Konzentration des Gases oder Dampfes unterhalb eines vorbestimmten Wertes liegt.

## Revendications

1. Appareil de stérilisation et/ou de désorption comprenant :
- une chambre de traitement (10) ;
- un moyen pour libérer un gaz stérilisant ou une vapeur stérilisante dans ladite chambre de traitement (10) ;
- un moyen (29) pour évacuer ledit gaz stérilisant ou ladite vapeur stérilisante de ladite chambre de traitement (10) ;
- un moyen d'échantillonnage (24/81) pour échantillonner l'atmosphère à l'intérieur de ladite chambre de traitement (10), et comprenant :
- un moyen (80, 81) pour exposer ledit échantillon d'atmosphère à une radiation hyperfréquence, et
- un moyen (83) sensible à l'absorption de radiation hyperfréquence par ledit échantillon pour déterminer la présence et/ou la concentration de gaz ou de vapeur dans ledit échantillon ;
caractérisé par le fait que ledit appareil comprend en outre un moyen de commande (130) sensible à ladite présence et/ou concentration déterminée de gaz ou de vapeur pour commander au moins l'un parmi ledit moyen de libération et ledit moyen d'évacuation.

2. Appareil de stérilisation et/ou de désorption selon la revendication 1, comprenant en outre un moyen de passage (82) connectant ladite chambre de traitement (10) et ledit moyen d'échantillonnage (24) et à travers lequel l'atmosphère peut passer de la chambre de traitement (10) audit moyen d'échantillonnage (24).

3. Appareil de stérilisation et/ou de désorption selon la revendication 2, dans lequel ledit moyen de passage (82) comprend un moyen pour amener l'atmosphère provenant de ladite chambre de traitement (10) à circuler à travers ledit moyen d'échantillonnage (24).

4. Appareil de stérilisation et/ou de désorption selon l'une quelconque des revendications précédentes, dans lequel ledit appareil de stérilisation comprend un moyen de verrouillage et ledit moyen de commande (130) est actionnable pour permettre à ladite chambre de traitement d'être ouverte seulement lorsque la concentration dudit gaz ou de ladite vapeur se trouve au-dessous d'un niveau donné, tel que déterminé par ledit moyen d'échantillonnage.

5. Appareil de stérilisation et/ou de désorption selon l'une quelconque des revendications précédentes, dans lequel ledit moyen de commande est actionnable pour commander ledit moyen de libération en réponse audit moyen d'échantillonnage afin de contrôler la quantité de gaz ou de vapeur libérée dans ladite chambre de traitement, permettant ainsi de limiter la concentration dudit gaz ou de ladite vapeur dans ladite chambre de traitement.

6. Appareil de stérilisation et/ou de désorption selon l'une quelconque des revendications précédentes, dans lequel ledit moyen de commande est actionnable pour surveiller la concentration dudit gaz ou de ladite vapeur dans ladite chambre de traitement pendant ladite étape de désorption, et pour commander ledit moyen de propagation afin de contrôler la période d'irradiation conformément à la concentration détectée dudit gaz ou de ladite vapeur.

7. Appareil de stérilisation et/ou de désorption selon l'une quelconque des revendications précédentes, dans lequel ledit moyen d'échantillonnage comprend une chambre de détection dans laquelle un échantillon de ladite atmosphère de chambre est introduit, un moyen de propagation d'une radiation hyperfréquence dans la chambre de détection et un moyen pour faire varier les fréquences de rotation moléculaire quantummécanique de l'analyte, la fréquence de la source de micro-ondes et la fréquence de résonance de la chambre de détection de telle sorte qu'elles soient en résonance, déterminant de cette façon la présence et/ou la concentration dudit gaz ou de ladite vapeur dans ladite chambre de traitement.

8. Procédé de stérilisation et/ou de désorption d'articles, ledit procédé comprenant les opérations consistant à :
- placer lesdits articles dans une chambre de traitement (10) ;
- libérer un gaz stérilisant ou une vapeur stérilisante dans ladite chambre de traitement (10) ;
- par la suite évacuer au moins une partie dudit gaz stérilisant ou de ladite vapeur stérilisante de ladite chambre de traitement(10) ; et
- échantillonner l'atmosphère à l'intérieur de ladite chambre de traitement (10) par exposition d'un échantillon d'atmosphère à une radiation hyperfréquence et déterminer la présence et/ou la concentration de gaz et de vapeur dans ledit échantillon sur la base de l'absorption de la radiation hyperfréquence par ledit échantillon,
caractérisé par le fait que ledit procédé comprend en outre la commande d'au moins l'une parmi ladite libération et ladite évacuation conformément à la présence et/ou concentration déterminée dudit gaz ou de ladite vapeur.

9. Procédé selon la revendication 8, qui comprend en outre l'opération consistant à amener l'atmosphère provenant de ladite chambre de traitement (10) à circuler à travers ledit moyen d'échantillonnage (24).

10. Procédé selon l'une des revendications 8 ou 9, qui comprend en outre l'opération consistant à empêcher l'accès à ladite chambre de traitement (10) jusqu'à ce que la concentration dudit gaz ou de ladite vapeur soit au-dessous d'un niveau préétabli.
